# EUROPEAN PATENT APPLICATION

(11) **EP 3 502 679 A1**
(43) Date of publication of application: **26.06.2019**
(21) Application number: 17840640.1
(22) Date of filing: 23.02.2017
(51) Int. Cl.: G01N 27/22, G01K 13/02

(54) **EQUIPMENT FOR CONTROLLING AND MEASURING HUMIDITY IN GRAIN DRYERS**

(30) Priority: 19.08.2016 BR 202016019177 U
(71) Applicant: Baumgarten, Julio Carlos Benjamin, CEP 96050-460 Pelotas (BR)
(72) Inventor: Baumgarten, Julio Carlos Benjamin, CEP 96050-460 Pelotas (BR)
(74) Representative: Pereira Garcia, João Luís
(86) International application number: PCT/BR2017/050040
(87) International publication number: WO 2018/032070

(57) **Abstract**

An equipment for grain moisture control and measurement in grain dryers is the object described in this invention has the objective of solving the inconvenient described in the state of the technique, through an equipment with an capacitive sensor (15) that promotes the readings of the moisture of the grains in a precise and reliable manner, through pre-determined samples sizes and measurement intervals. With this technique, the process is fully automated, so there are no external interferences to be accounted, resulting in the best possible measurement, as well as the best possible outcome possible to the grain's quality.

## Description

This invention describes an equipment for moisture measurement and control in grain dryers. More specifically, it is composed of a capacitive sensor that provides the value of the grain moisture through the measuring of a pre-defined product volume, eliminating the possibility of external interference, thus ensuring highly precise and reliable readings.

The procedures for grain preservation comprehend multiple stages, from harvesting to storage. The determination of the moisture percentage is of utmost importance while handling the grain, to ensure the right course of action is being taken. Thus, avoiding significant changes to the grain's physical and nutritional value, as well as its commercial value, especially during its storage.

The moisture of the grain is one of the most important values to be controlled in the storage process, directly affecting the quality, as well as the time it can be stored. The deterioration of the grain due to insects, fungi, as well as the rate at which chemical and enzymatic processes occur, are directly connected to the moisture. So, it is of utmost importance to have fast and accurate measurements since the harvest, to ensure the product's quality.

The methods utilized to determine the moisture are divided in two categories: direct and indirect. The direct methods determine the quantity of moisture in the grain by removing the water from it, while the indirect ones utilize the electric properties of the grain, like capacitance or resistance.

The harvested grains present a moisture too high to be stored, so the grain passes through a drying process, until the grain reaches 13% moisture, which is considered a safe value for storage. Real time monitoring of the moisture is necessary to ensure the grain is being well stored.

The direct methods, which generally are more precise, however, need a longer period to determine the moisture, such as the official greenhouse method, that takes as much as 24 hours to obtain these results. The indirect methods are faster, since the moisture percentage is estimated as a function of the electric properties of the product, but they are prone to errors due to variations of physical characteristics of the grain, such as temperature and the moisture range.

The state of the technique presents several documents that claim moisture measuring devices, in which can be highlighted the following documents:
The document BRPI0905021-3 refers to a process and an equipment for moisture measurement within a grain sample which applies the principles of distillation. The equipment used to determine the moisture within a grain sample contains a container with a lid and an instrument to measure the internal temperature. This container then receives a pre-determined mass of grain and an adequate amount of oil. A pipeline then connects the container and a condenser. A heat source is applied to the container. A graded container is used to collect and measure the volume of water that evaporated off the sample and subsequently condensed.

This document referred to the state of the technique presents a grain moisture measurement device which uses the principle of distillation to measure the moisture and control the process. This method, however, does not provide precise readings and reliable results for the measures. However, the equipment claimed in this patent requisition describes a new constructive disposition which allows the control and measurement automatically, in precise and reliable manner.

The document BRPI1106740-3 refers to a sample collector utilizing an elevator, a level sensor at the measuring cell, a sensor to avoid overfilling the container. This system executes readings with static samples and can be placed at any point in the process.

This document referred to the state of the technique presents an equipment for measuring the grain moisture similar to the equipment being claimed in this patent requisition, with the present request having a new constructive disposition, which allows for better control and measurement precision and reliability.

The object in the present utility model is an equipment of control and measurement of the grain's moisture in grain dryers, which presents a capacitive sensor to promote the readings of the moisture in the grain, through a pre-determined volume of product, this way, controlling the moisture according to the value read with the equipment mentioned. Thus, ensuring the reliability of the product, for it is completely automated.

These characteristics make the referred equipment in this patent requisition more precise and reliable when compared to the current state of the technique.

It is a characteristic of invention an equipment for measurement and control of grain moisture that provides a constructive disposition that allows the definition of a pre-defined volume as a parameter for measurements.

It is a characteristic of invention an equipment for measurement and control of grain moisture that provides a capacitive sensor which measures the moisture and temperature of the grain, which may vary according to the type of grain.

It is a characteristic of invention an equipment for measurement and control of grain moisture that provides a mobile wall to compress the grains, which is actuated by a pneumatic cylinder.

It is a characteristic of invention an equipment for measurement and control of grain moisture that allows the adjustment of pressure for the compressing cylinder, so that the different textures of grains do not interfere in the process.

It is a characteristic of invention an equipment for measurement and control of grain moisture that provides a pneumatic cylinder underneath the equipment to stop the flow of grain, in order to acquire a sample for the measurement process.

In order to describe the technical characteristics if the equipment for control and measurement of grain moisture in grain dryers, the following pictures are presented in this document:
Figure #1 presents the perspective view of the equipment.
Figure #2 presents the lateral view of the equipment.
Figure #3 presents the frontal view of the equipment.
Figure #4 presents the posterior perspective, without the protections attached.
Figure #5 displays the perspective view of the equipment, detailing the actuating components of the cylinder.
Figure #6 presents the perspective view of the equipment, detailing the sensors disposed at the side.
Figure #7 presents the flow chart of the micro controlled system of the equipment, where "X" represents the number of samples and "Y" the necessary amount of samples, in order to measure and control the moisture in the grain.

The equipment for control and measurement of grain moisture in grain dryers, object of the current invention, comprehends one structure (10) containing at the superior portion an input hole for the grain (11), while at the bottom part there is an output hole (12) for the grains that go through the measuring process.

The structure (10) describes internally a mobile wall (13) which is actuated by a pneumatic cylinder (14), pressuring the grain against the structure (10) to ensure lesser air in between the grain, and a more uniform mass of grain.

The structure (10) describes the disposition of a capacitive sensor (15), positioned in from of the grain mass that is being pressured by the mobile wall (13), to measure the amount of water in the sample.

The mobile wall (13) is actuated by a pneumatic cylinder (14) interconnected to an arm (141) which transmits the movement to a mechanism (131) of actuation of the mobile wall (13), with the pneumatic cylinder (14) being controlled by the micro processed system.

The structure (10) presents a temperature sensor (18) located at the frontal face of the equipment, as well as a level sensor (19) located at the side of the equipment, close to the input hole (11).

The temperature sensor (19) informs the micro processed system the temperature of the sample, helping in the process of detecting the moisture, along with the capacitive sensor (15) .

The level sensor (19) informs the system that the grains are being accumulated inside the structure (10), allowing the moving wall (13) to be actuated in order to measure the moisture.

The output hole (12) has a gate (16) actuated by a cylinder (17), which closes the flow of grain through the equipment, in order for it to be stored inside the structure (10). This occurs in a fully automated manner, according to the pre-determined time set to the system, which may be changed according to the situation.

The equipment reaches peak condition for measurement of grain moisture by defining the size of the structure (10) which will then present a pre-defined volume for measurement, ensuring the grain mass have a uniform distribution on the surface of the capacitive sensor (15).

The fixed space on the structure (10) is obtained by the compression of the sample against a movable wall (13), actuated by a pneumatic cylinder (14). The pressure made by the wall (13) is adjusted to ensure the uniformity of the sample so that the air spaces in between the grains does not affect the measurement.

At the same time the capacitive sensor (15) makes the measurement, the temperature is also being measured, through a temperature sensor (18), which is in the same chamber in the structure (10). With this data, it is possible to calculate the moisture of the grain, after its hydroscopic balance. This information is vital, since this is the most accurate value, instead of the instantaneous value.

These calculations are made with equations generated through technology on grain conservation, possessing various complex tables that are used to reach the final value.

The calculation is made via a micro processed equipment, which receives the signals and make the due treatment to the signal. The values read and treated are then run through technical tables that correlate the values obtained with grain quality and conservation values. These numbers are final qualitative values used to take decisions in order to obtain the best product possible.

The capacitive sensor (15) utilized measures the capacitance of the sample, which varies according to the quantity of water on the sample and the type of grain. For each grain, a calibration must be performed, which will then be considered the default configuration. The dielectric constant of the sample varies according to the quantity of water in it, which is directly related to the capacitance, and it can be correlated in a way that the system has a characteristic curve with all the moisture percentage x capacitance points, inside a determined range.

The capacitive sensor (15) utilizes as output a 4-20mA signal, which means that when the sensor is providing 4mA it will be at its lowest value, and 20mA its maximum value according to the characteristic curve.

The minimum and maximum values are determined by the moisture to be read, usually being around 10% and 25%. Once the capacitance values are available, they can be read electronically via micro processed systems, which will perform the necessary calculations to convert the capacitance read into a moisture percentage. Inside this micro processed system will be all the physics and mathematics utilized to calculate the moisture of the grain at its hygroscopic equilibrium.

The micro processed equipment that makes all the readings, operations and signal treatment is located in a metallic box, next to the moisture measuring device. This equipment possesses the capacity to control all motors and actuators of the grain dryers, ensuring the possibility of reaching the desired quality. In order to reach a perfect control system, it needs to be connected to the industrial automation. To do that, it is necessary to have a communication port in the device, which will deliver the data accordingly.

The equipment presents the disposition of protection structures for sensors (20) and pneumatic cylinders (14) (17), in order to protect these components from damage due to mishandling. This way, the current invention describes an equipment that has a capacitive sensor to provide the measurement of the grain moisture through a pre-determined sample size in specified intervals. This ensures a more precise and trustworthy outcome, due to the process being completely automated, eliminating external interferences.

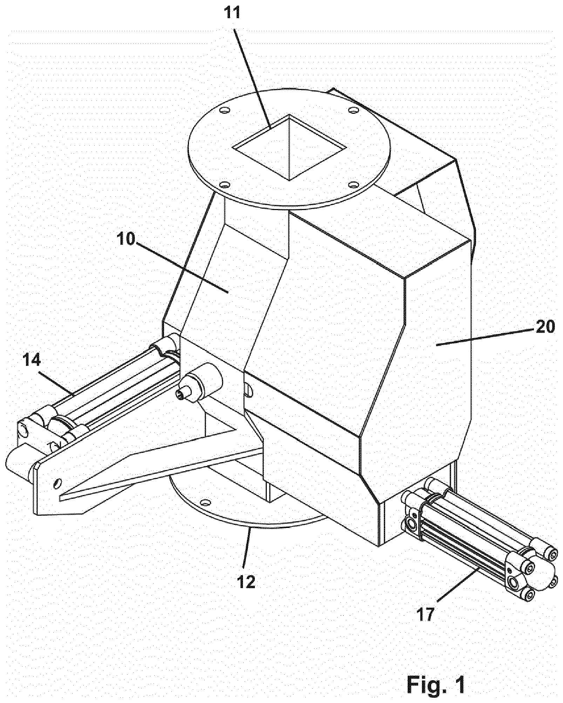

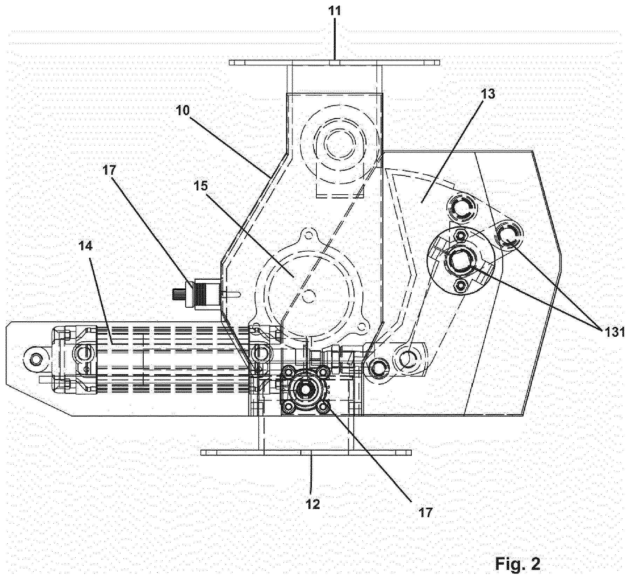

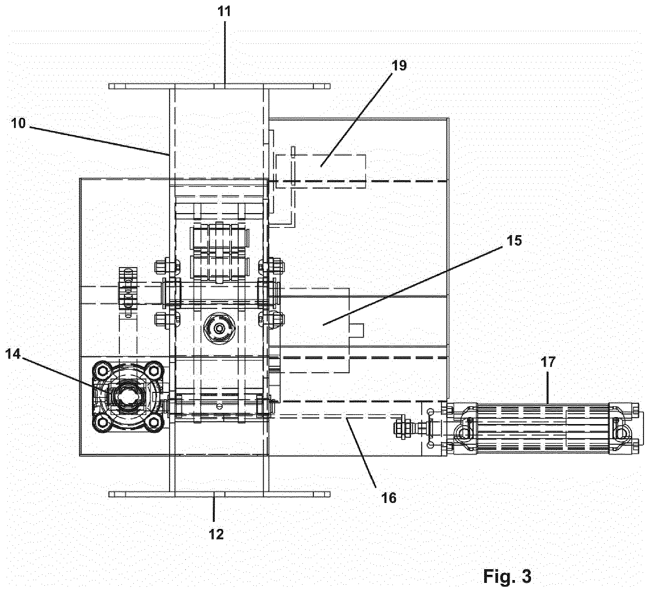

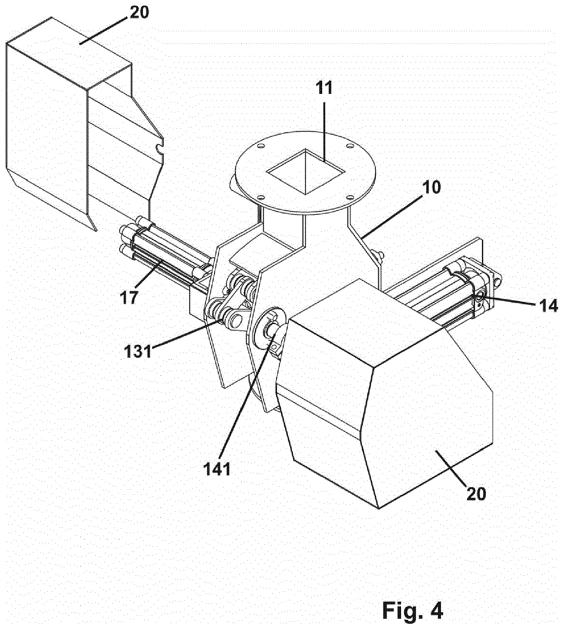

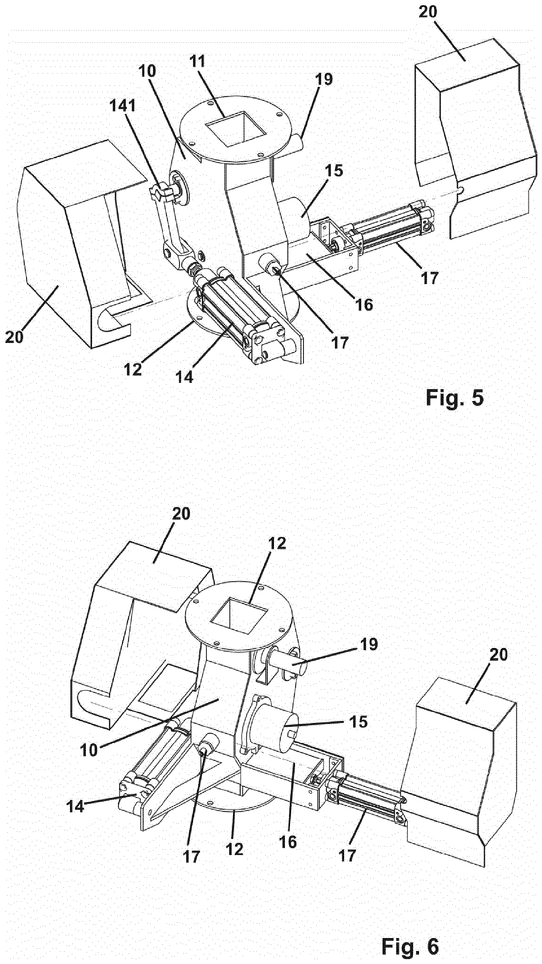

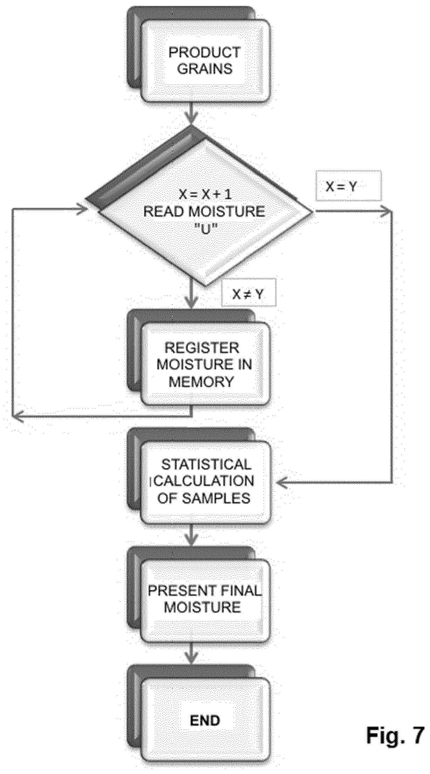

## Claims

1. EQUIPMENT FOR GRAIN MOISTURE CONTROL AND MEASUREMENT IN GRAIN DRYERS **characterized by** comprehending a structure (10) with an input hole (11) in the superior part, and an output hole (12) in the bottom part. The structure (10) describes, internally, a chamber where a mobile wall (13), actuated by a pneumatic cylinder (14) interconnected to an arm (141) which transmits the movement to the mechanism (131) of the mobile wall (13), with this pneumatic cylinder (14) being controlled by the micro processing system; The structure (10) describes the disposition of a capacitive sensor (15) positioned in front of the grain mass that is being pressed by the mobile wall (13) to measure the quantity of water in the sample, a temperature sensor (18) located at the front of the equipment, and a level sensor(19) close to the input hole (19) at the side of the equipment. The output hole (12) presents a gate (16) actuated by a pneumatic cylinder (17), which closes the flow of product, to accumulate the product inside the structure (10), with all the equipment being controlled by the micro processed system.

2. EQUIPMENT FOR GRAIN MOISTURE CONTROL AND MEASUREMENT IN GRAIN DRYERS, according to the claim 1, **characterized by** the temperature sensor (18) to help the moisture calculations, together with the capacitive sensor (15).

3. EQUIPMENT FOR GRAIN MOISTURE CONTROL AND MEASUREMENT IN GRAIN DRYERS, according to the claim 1, **characterized by** a level sensor (19) to inform the micro processed system that the grain is inside the structure (10), allowing the mobile wall (13) to be actuated to press the grain inside.

4. EQUIPMENT FOR GRAIN MOISTURE CONTROL AND MEASUREMENT IN GRAIN DRYERS, according to the claim 1, **characterized by** the structure (10) presenting a pre-determined volume for measuring.

5. EQUIPMENT FOR GRAIN MOISTURE CONTROL AND MEASUREMENT IN GRAIN DRYERS, according to the claim 1, **characterized by** the pressure made by the movable wall (13) being adjusted to ensure high uniformity and less air in the grain mass.

6. EQUIPMENT FOR GRAIN MOISTURE CONTROL AND MEASUREMENT IN GRAIN DRYERS, according to the claim 1, **characterized by** the calculations being made by a micro processed system, which receives the signals and provides its due treatment. The values read and treated are then run through technical tables that correlate the values obtained with grain quality and conservation values. These numbers are final qualitative values used to take decisions in order to obtain the best product possible.

7. EQUIPMENT FOR GRAIN MOISTURE CONTROL AND MEASUREMENT IN GRAIN DRYERS, according to the claim 1, **characterized by** the capacitive sensor (15) that utilizes as output a 4-20mA signal, which means that when the sensor is providing 4mA it will be at its lowest value, and 20mA its maximum value according to the characteristic curve.

8. EQUIPMENT FOR GRAIN MOISTURE CONTROL AND MEASUREMENT IN GRAIN DRYERS, according to the claim 1, **characterized by** the equipment presenting the disposition of protection structures for sensors (20) and pneumatic cylinders (14) (17), in order to protect these components from damage due to mishandling. This way, the current model of utility describes an equipment that has a capacitive sensor to provide the measurement of the grain moisture through a pre-determined sample size in specified intervals. This ensures a more precise and trustworthy outcome, due to the process being completely automated, eliminating external interferences.
